# EUROPEAN PATENT SPECIFICATION

(11) **EP 2 124 858 B1**
(45) Date of publication and mention of the grant of the patent: **04.05.2011**
(21) Application number: 08720245.3
(22) Date of filing: 18.02.2008
(51) Int. Cl.: A61F 13/00

(54) **AUXILIARY DEVICE FOR WEARING A TERAPEUTIC ELASTIC STOCKING**
HILFSGERÄT ZUM TRAGEN THERAPEUTISCHER ELASTISCHER STRÜMPFE
DISPOSITIF AUXILIAIRE POUR PORTER UN BAS ÉLASTIQUE THÉRAPEUTIQUE

(30) Priority: 19.02.2007 IT RM20070089
(43) Date of publication of application: 02.12.2009
(73) Proprietor: Varimed S.r.l., 95123 Catania (IT)
(72) Inventor: MARINO, Roberto, I-95123 Catania (IT)
(74) Representative: Gitto, Serena
(86) International application number: PCT/IT2008/000101
(87) International publication number: WO 2008/102398

(56) References cited:
- WO-A-99/44548
- GB-A- 2 316 600
- US-A- 5 356 057
- US-B1- 6 523 729

## Description

The present invention relates to an auxiliary device for wearing therapeutic elastic stocking.

More specifically, the invention relates to a device permitting a quick and intuitive wearing of a therapeutic stocking on a limb, aiding users having some motion problems.

As it is well known, after surgical interventions for different kinds of pathologies, elastic stockings are often used, these mainly comprised of synthetic fibres, said stocking having high retention properties, suitable to be applied to the concerned limbs, such as a forearm, a foot or a leg.

Said stockings are used in rehabilitative medicine. For example, they are necessary for post-surgical convalescences, such as post-surgical rehabilitation of knee arthroprosthesis.

Furthermore, their use is strongly advised after a deep venous thrombosis, since they can reduce the risk of post-thrombosis complications.

A problem connected with elastic stockings is that they are usually difficult to be put on just because of their high retention capabilities. This problem is particularly important for elderly patients, mainly for their reduced mobility.

Auxiliary devices have been available on the market since long time heeping the user in the wearing of said retention elastic stockings on the interested limb.

One kind of device available since long time on the market is comprised of natural fibre-slippery tissue comprised of two fabric pieces partially coupled along their edge, so as to realise something similar to "shippers".

For wearing the elastic stocking the user must first weave the above mentioned device. Thanks to the slippery material, it is possible to easily weare the stocking and then to remove the same device following its traction by the same user by traction of the top.

A problem related to the known devices is that often, particularly for an old person, and if the concerned limb is a foot, it is not easy grasping the tip and taking away the device. In fact, contemporaneous sliding between device and user skin during the withdrawal phase often has a remarkable resistance.

In view of the above mentioned problems, it is object of the present invention that of suggesting a device that can be used in an intuitive way and does not require any preparation by the user before being put on.

It is therefore specific object of the present invention an auxiliary device for wearing an elastic stocking comprising a first piece of tissue, comprised of slippery material; and a second piece of tissue, comprised of slippery material, coupled along a first portion of its edge with a corresponding first portion of the edge of said first piece of tissue, so as to realize an funnel shaped pocket, closed on its bottom; characterized in that said first and second pieces of tissue respectively comprise a first and a second guide slot, placed in correspondence of said pocket bottom; in that it comprises a band having a first end fixed in correspondence of the free portion of said edge of said first piece of tissue and a second end fixed in correspondence of the free portion of said edge of the second piece of tissue, said band passing through said first and second guide slots and having such a length to realize a ring in correspondence of said slots, said ring being usable for grasping when said device and said elastic stocking are put on, for pulling, folding and sliding each other said first and second pieces of tissue, thus easing extraction of the same device on said stocking; and in that said first piece of tissue is comprised of high density polyethylene and/or nylon and/or tissue and/or synthetic thermoplastic material and/or tetrafluorethylene, polyethylene and/or glass wool, and said second piece of tissue is comprised of high density polyethylene and/or nylon and/or tissue and/or synthetic thermoplastic material and/or tetrafluorethylene, polyethylene and/or glass wool.

Always according to the invention, said first and said second pieces of tissue can be coupled by stitching.

Still according to the invention, said second piece of tissue has a surface larger with respect to said first piece of tissue.

The present invention will be now described, for illustrative but not limitative purposes, according to its preferred embodiments, with particular reference to the figures of the enclosed drawings, wherein:
figure 1 shows a top plan view of the auxiliary for wearing a therapeutic stocking according to the present invention;
figure 2 shows a bottom plan view of the device of figure 1;
figure 3 shows a perspective view of the device according to figure 1;
figure 4 shows a lateral view of the device according to figure 1 put on a user;
figure 5 shows a lateral view of device of figure 1 put on a user, with an elastic stocking while is being put on the device;
figure 6 shows a lateral view of device of figure 1 put on a user, with an elastic stocking completely being put on the device; and
figure 7 shows a bottom plan view of device according to figure 1 with a piece of fabric folded.

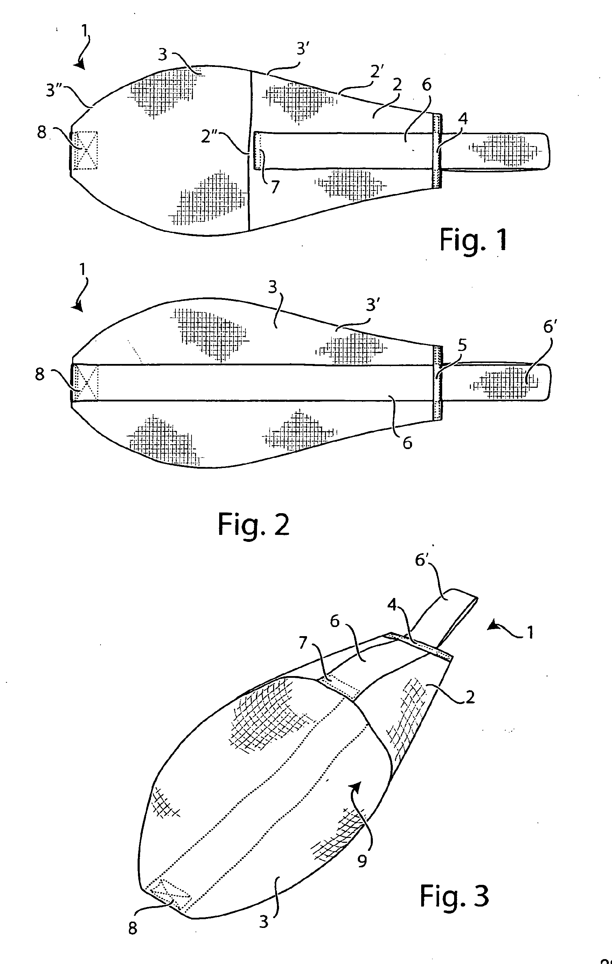

Making reference to figures 1, 2 and 3, it is possible to observe auxiliary device 1 for wearing a therapeutic stocking according to the invention.

It is noted that the device 1 includes a first piece of tissue 2, with its edge comprised of a first 2' and a second 2" portion, and a second piece of tissue 3, having its edge comprised of a first 3' and a second 3" portion.

Said first piece of tissue 2 is stitched along said first portion 2' of the edge with said second piece of tissue 3 along said first portion 3' of the edge.

From figure 3, it is noted that said first and second pieces of tissues 2, 3 are stitched along relevant portions 2', 3' of the edge, realising a kind of pocket 9 or division, within which, in an operative configuration, the limb can be introduced on which the elastic stocking (not shown in the figure) must be put on.

Said pocket 9 has a closed funnel shape with its bottom stitched.

Said first and second pieces of fabric 2, 3 comprise two guide slots, respectively indicated by reference numbers 4 and 5. Said guide slots 4 and 5 are juxtaposed and provided in correspondence of the bottom of the pocket 9, on its lateral edges 2', 3'.

Device 1 is also provided with a band 6, with a first end 7 stitched on said first piece of fabric 2, in correspondence to the free portion 2" of its edge, while second end 8 is stitched on said second piece of fabric 3 in correspondence to the free portion 3" of its edge.

Said band 6 passes through both said guide slots 4, 5. The length of said band 6 is such to realise with its exceeding part, a handle 6' in correspondence of said guide slots 4, 5.

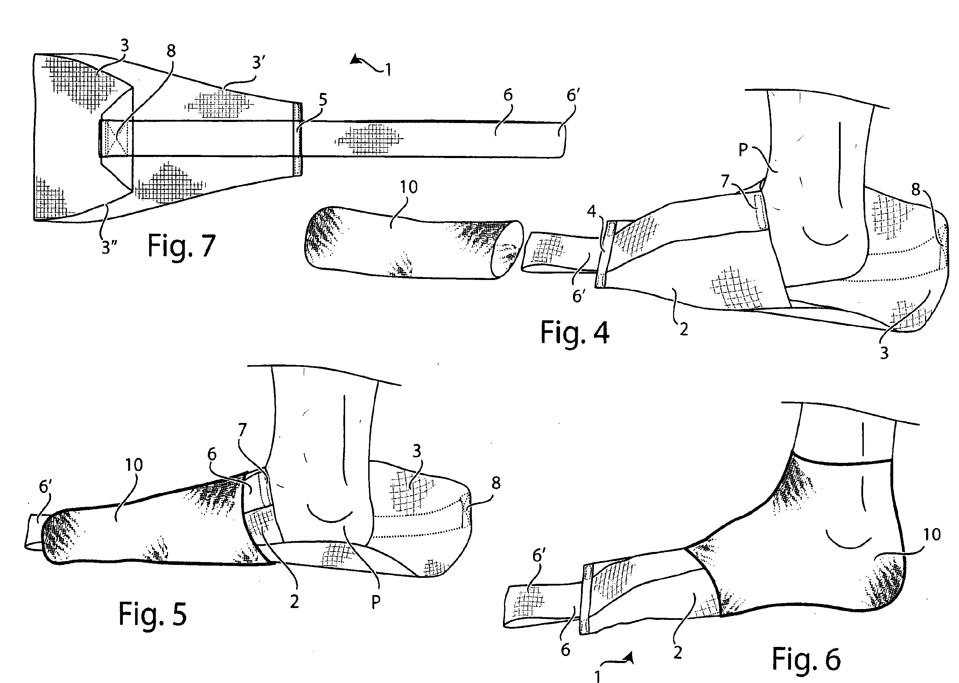

Figures 4, 5 and 6 show operation of device 1 for easing the work of a user to put on an elastic stocking, said stocking being indicated in the figures by reference number 10. The device 1 is shown in its embodiment for putting on an elastic stocking on a foot or on a leg, but the following specification is valid also for putting on elastic stockings 10 on other limbs, by suitably varying dimensions of the device 1 and of the elastic stocking 10.

As it can be observed, the user simply introduces the foot P within the pocket 9 realised between said first and second pieces 2, 3 of fabric.

It is clearly noted that:
- the device 1 does not require a preparation;
- the introduction of the limb (in this case of a foot) within the device 1, and particularly within the pocket 9, is a very natural act.

When the device 1 is put on by a user it is possible to easily put on the elastic stocking 10.

Once having put on the elastic stocking 10, being the latter realised in a tubular shape, the device permits easy grasping the part of the band 6 comprising a grasping piece of tissue 6'.

Then, for removal of said device 1, that now is between the elastic stocking 10 and the limb, i.e. the foot P or the leg, it is sufficient pulling the grasping piece of tissue 6' of the band 6.

Said band 6 will make at the same time a traction on both portions 2", 3" of the edges of said first and second pieces of tissue 2, 3, that, as it can be observed, are, the first one in correspondence of the feet back, and the second one under the feet sole.

Both tissue pieces, after traction of the band 6, are folded, thus sliding and making removal of device 1 easier. This effect is particularly airlined in figure 7 for the second piece 3 of fabric, showing that it is folded due to the traction by the band 6.

Device 1 is comprised of low friction synthetic material. Anyway, said device 1 can be comprised of different and materials for each piece of tissue 2, 3, among which nylon, synthetic thermoplastic material film or tissue, tetrafluoroethylene, poli-tetrafluoroethylene, glass wool.

On the basis of the above specification, it can be observed that a major advantage of the present invention is that of being usable in a very easy and intuitive way.

A further advantage of the solution according to the present invention is that of being very economic to be manufactured, since it has a very simple structure requiring few materials.

The present invention has been described for illustrative but not limitative purposes, according to its preferred embodiments, but it is to be understood that modifications and/or changes can be introduced by those skilled in the art without departing from the relevant scope as defined in the enclosed claims.

## Claims

1. Auxiliary device (1) for wearing an elastic stocking (10) comprising
a first piece of tissue (2), comprised of slippery material; and
a second piece of tissue (3), comprised of slippery material, coupled along a first portion (3') of its edge with a corresponding first portion (2') of the edge of said first piece of tissue (2), so as to realize a funnel shaped pocket (9), closed on its bottom;
**characterized in that**
said first (2) and second (3) pieces of tissue respectively comprise a first (4) and a second (5) guide slot, placed in correspondence of said pocket (9) bottom;
**in that** it comprises a band (6) having a first end (7) fixed in correspondence of the free portion (2") of said edge of said first piece of tissue (2) and a second end (8) fixed in correspondence of the free portion (3") of said edge of the second piece of tissue (3), said band (6) passing through said first and second guide slots (4, 5) and having such a length to realize a ring (6') in correspondence of said slots (4, 5), said ring (6') being usable for grasping when said device (1) and said elastic stocking (10) are put on, for pulling, folding and sliding each other said first (2) and second (3) pieces of tissue, thus easing extraction of the same device (1) on said stocking (10); and
**in that** said first piece of tissue (2) is comprised of high density polyethylene and/or nylon and/or tissue and/or synthetic thermoplastic material and/or tetrafluorethylene, polyethylene and/or glass wool, and said second piece of tissue (3) is comprised of high density polyethylene and/or nylon and/or tissue and/or synthetic thermoplastic material and/or tetrafluorethylene, polyethylene and/or glass wool.

2. Device (1) according to claim 1, **characterized in that** said first (2) and said second (3) pieces of tissue can be coupled by stitching.

3. Device (1) according to one of the preceding claims, **characterized in that** said second piece (3) of tissue has a surface larger with respect to said first piece (2) of tissue.

## Patentansprüche

1. Hilfsvorrichtung (1) zum Antragen eines elastischen Strumpfs (10), umfassend:
einen ersten Gewebeteil (2), umfassend gleitfähiges Material; und
einen zweiten Gewebeteil (3), umfassend gleitfähiges Material, gekoppelt entlang eines ersten Abschnitts (3') seiner Kante mit einem entsprechenden ersten Abschnitt (2') der Kante des ersten Gewebeteils (2), um eine trichterförmige Tasche (9) zu realisieren, die an ihrem Boden geschlossen ist;
**dadurch gekennzeichnet, dass**
der erste (2) und zweite (3) Gewebeteil einen ersten (4) beziehungsweise einen zweiten (5) Führungsschlitz umfassen, entsprechend positioniert zu dem Boden der Tasche (9);
dass diese ein Band (6) umfasst, aufweisend ein erstes Ende (7), fixiert entsprechend dem freien Abschnitt (2") der Kante des ersten Gewebeteils (2), und ein zweites Ende (8), fixiert entsprechend dem freien Abschnitt (3") der Kante des zweiten Gewebeteils (3), wobei das Band (6) durch den ersten und zweiten Führungsschlitz (4, 5) tritt und eine derartige Länge aufweist, um einen Ring (6') entsprechend den Schlitzen (4, 5) zu realisieren, wobei der Ring (6') verwendbar ist zum Greifen, wenn die Vorrichtung (1) und der elastische Strumpf (10) angezogen sind, zum Ziehen, Falten und Gleiten jeweils des ersten (2) und zweiten (3) Gewebeteils, um das Entfernen derselben Vorrichtung (1) an dem Strumpf (10) zu erleichtern; und
dass der erste Gewebeteil (2) ein eine hohe Dichte aufweisendes Polyethylen umfasst und/oder Nylon und/oder Gewebe und/oder synthetisches thermoplastisches Material und/oder Tetrafluorethylen, Polyethylen und/oder Glaswolle, und der zweite Gewebeteil (3) ein eine hohe Dichte aufweisendes Polyethylen umfasst und/oder Nylon und/oder Gewebe und/oder synthetisches thermoplastisches Material und/oder Tetrafluorethylen, Polyethylen und/oder Glaswolle.

2. Vorrichtung (1) nach Anspruch 1, **dadurch gekennzeichnet, dass** der erste (2) und der zweite (3) Gewebeteil durch Vernähen gekoppelt sind.

3. Vorrichtung (1) nach einem der vorhergehenden Ansprüche, **dadurch gekennzeichnet, dass** der zweite Gewebeteil (3) in Bezug auf den ersten (2) Gewebeteil eine größere Oberfläche aufweist.

## Revendications

1. Dispositif auxiliaire (1) pour le port d'un bas élastique (10) comprenant
un premier morceau de tissu (2), composé de matière glissante ; et un second morceau de tissu (3), composé de matière glissante, associé le long d'une première partie (3') de son bord à une première partie correspondante (2') du bord dudit premier morceau de tissu (2), de façon à réaliser une poche en forme d'entonnoir (9), fermée en sa partie inférieure ;
**caractérisé en ce que**
lesdits premier (2) et second (3) morceaux de tissu comprennent respectivement une première (4) et une seconde (5) fentes de guidage, placées en correspondance de la partie inférieure de ladite poche (9) ;
**en ce qu'**il comprend une bande (6) comportant une première extrémité (7) fixée en correspondance de la partie libre (2") dudit bord dudit premier morceau de tissu (2) et une seconde extrémité (8) fixée en correspondance de la partie libre (3") dudit bord du second morceau de tissu (3), ladite bande (6) passant à travers lesdites première et seconde fentes de guidage (4, 5) et ayant une longueur propre à permettre la réalisation d'un anneau (6') en correspondance desdites fentes (4, 5), ledit anneau (6') pouvant être saisi lorsque l'on enfile ledit dispositif (1) et ledit bas élastique (10), pour tirer, plier et faire glisser mutuellement lesdits premier (2) et second (3) morceaux de tissu, en facilitant ainsi l'extraction de ce même dispositif (1) sur ledit bas (10) ; et
**en ce que** ledit premier morceau de tissu (2) est composé de polyéthylène haute densité et/ou de nylon et/ou de tissu et/ou de matière thermoplastique synthétique et/ou de tétrafluoroéthylène, de polyéthylène et/ou de laine de verre, et ledit second morceau de tissu (3) est composé de polyéthylène haute densité et/ou de nylon et/ou de tissu et/ou de matière thermoplastique synthétique et/ou de tétrafluoroéthylène, de polyéthylène et/ou de laine de verre.

2. Dispositif (1) selon la revendication 1, **caractérisé en ce que** ledit premier (2) et ledit second (3) morceaux de tissu peuvent être associés en les cousant.

3. Dispositif (1) selon l'une quelconque des revendications précédentes, **caractérisé en ce que** ledit second morceau (3) de tissu comporte une surface plus large par rapport audit premier morceau (2) de tissu.
